# EUROPÄISCHE PATENTSCHRIFT

(11) **EP 3 622 289 B1**
(45) Veröffentlichungstag und Bekanntmachung des Hinweises auf die Patenterteilung: **23.08.2023**
(21) Anmeldenummer: 18725797.7
(22) Anmeldetag: 11.05.2018
(51) Int. Cl.: G01N 33/48, A61B 10/00

(54) **VERFAHREN UND TESTBESTECK ZUR QUANTITATIVEN BESTIMMUNG VON BIOMARKERN IN FÄKALPROBEN**
METHOD AND KIT FOR THE QUANTITATIVE DETERMINATION OF BIOMARKERS IN FAECAL SAMPLES
PROCÉDÉ ET KIT POUR DÉTERMINER QUANTITATIVEMENT DES BIOMARQUEURS DANS DES ÉCHANTILLONS FÉCALS

(30) Priorität: 11.05.2017 DE 102017110294
(43) Veröffentlichungstag der Anmeldung: 18.03.2020
(73) Patentinhaber: Immundiagnostik AG, 64625 Bensheim (DE)
(72) Erfinder: GROEN, Hans-Juergen, 64625 Bensheim (DE); ARMBRUSTER, Franz-Paul, 64625 Bensheim (DE)
(74) Vertreter: Benedum, Ulrich Max
(86) Internationale Anmeldenummer: PCT/EP2018/062222
(87) Internationale Veröffentlichungsnummer: WO 2018/206772

(56) Entgegenhaltungen:
- WO-A1-2016/014822
- JP-A- 2003 014 768
- JP-A- 2003 194 825
- JP-B2- 6 077 763
- US-A- 5 094 956
- EDWARDS ET AL: "Improving fecal blood testing", GASTROENTEROLOGY, W.B. SAUNDERS CO, US, Bd. 114, Nr. 1, 1. Januar 1998 (1998-01-01), Seiten 226-227, XP005138351, ISSN: 0016-5085, DOI: 10.1016/S0016-5085(98)70662-6

## Beschreibung

### TECHNISCHES GEBIET

Die Erfindung betrifft Verfahren zur quantitativen Bestimmung von Biomarkern in fäkalen Proben sowie einen Bestimmungssatz hierfür.

### HINTERGRUND DER ANMELDUNG

Die klinische Untersuchung von Ausscheidungen des Darms ist unbeliebt. Zum einen kann der Arzt nicht sofort eine Probe nehmen, sondern er muss erst einen Stuhlgang der Person abwarten. Kot, Stuhl oder medizinisch Faeces ist unhygienisch, geruchsintensiv und ekelerregend. Daher wird dem Patienten eine Vorrichtung in die Hand gegeben, damit er selbst etwas von seinem Stuhl in ein Untersuchungsgefäß überführen kann - ein in Regel nicht kontrollierter Schritt. Für eine quantitative Bestimmungen muss die fäkale Probe zudem eingewogen oder volumenmäßig bestimmt werden. Diese ist aber nicht homogen, sondern Matrix, Konsistenz und Zusammensetzung hängen ab von der Nahrung und dem Gesundheitszustand. Die diagnostischen Zielproteine sind zudem nicht stabil. Darmbakterien und Verdauungsenzyme sind im Darm aktiv und sind es auch nach der Ausscheidung. Sie greifen nicht nur die Zielproteine an, sondern sie stören auch die enzymatische oder immunologische Bestimmung.

Fäkale Proben werden daher bis zur Untersuchung zumeist eingefroren. Das Einfrieren und Auftauen von Stuhl ist mit Stehzeiten verbunden, in denen Abbau und Zersetzung weiterlaufen. Für kleine Mengen gibt es auch Stabilisierungspuffer. Nachteilig daran ist, dass sich die Verdauungsprozesse nur schwer inhibieren lassen und die Stabilisierung im Puffer für mindestens fünf bis sieben Tage bei Umgebungstemperatur gut sein muss, denn solange es zwischen Probennahme und Untersuchung im Labor dauern. Für die Präanalytik von Stuhlproben gilt allgemein, dass der Analyt im Stabilisierungspuffer 5 bis 7 Tage ungekühlt überstehen muss, auch ein Wochenende, Feiertage und längere Transportprozesse, wobei die warme Jahreszeit und extreme Transportbedingungen berücksichtigt werden müssen.

Schließlich muss der Analyt aus der Stuhlmatrix extrahiert und in Lösung gebracht werden. Wird ein chaotropischer Puffer für die Extraktion verwendet, so müssen die chaotropischen Salze vor einer immunologischen Bestimmung entfernt werden, so dass der proteinöse Zielanalyt wieder renaturieren kann. Okkultes Blut bzw. Hämoglobin wird in konventionellen Puffersystemen zumeist mit einer Rate von 6 bis 7 Prozent pro Tag abgebaut (Grazzini et al, Influence of seasonal variations in ambient temperatures on performance of immunochemical faecal occult blood test for colorectal cancer screening: observational study from the Florence district, Gut (2010), 59(11): 1511-5*;* Van Room et al, Are fecal immunochemical test characteristics influenced by sample retum time? A population-based colorectal cancer screening trial. The American J. of Gastroenterology (2012) 107:99-107).

Getrocknete Stuhlproben werden auch auf besonderen Papierträgern (Whatman FTA^{®} bzw. FTA^{®} Card) konserviert und gelagert (Nechvatal JM et al in Fecal collection, ambient preservation, and DNA extraction for PCR amplification of bacterial and human markers from human faeces, J Microbiol Methods. (2008) 72(2):124-32; Chu DM, et al., Detection of Cyclospora cayetanensis in animal fecal isolates from Nepal using an FTA filter-base polymerase chain reaction method. Am J Trop Med Hyg. (2004) 71(4):373-9; Subrungniang I, et al., Evaluation of DNA extraction and PCR methods for detection of Enterocytozoon bienuesi in stool specimens J Clin Microbiol. (2004) 42(8):3490-4). WO2016014822 (GE Healthcare) offenbart Träger aus einem Fasermaterial wie Cellulose, Glasfaser oder Mikrofasern. Es werden auch poröse Membranen eingesetzt aus Polyester, Polyethersulfon (PES), Polyamid (Nylon), Polypropylen, Polytetrafluorethylen (PTFE), Polycarbonate, Cellulosenitrat, Celluloseacetat, oder Alginat. Die fäkale Probe wird zuweilen bei der Trocknung auf dem Träger auch mit chaotropen und denaturierenden Substanzen behandelt; siehe EP1 109899 (Whatman PLC). Der Analyt bei getrockneten Stuhlproben ist aber regelmäßig eine Nukleinsäure, die bspw. mit einer Kettenpolymerasereaktion (PCR) analysiert wird. Proteine entziehen sich einer quantitativen Bestimmung, denn sie denaturieren dabei und binden über hydrophobe Wechselwirkungen "nicht mehr ablösbar" am Träger. Eine differenzierende quantitative Analyse ist, wenn überhaupt, dann mit einfachen Mittel nicht mehr möglich.

JP2003 014768 A**,** JP2003 194825 A**,** JP 6 077763 B2 lehren Puffer und Verfahren zur Stabilisierung von Hämoglobin in Lösung für den Nachweis von okkultem Blut im Stuhl. Edwards et al. offenbaren in dem Artikel *Improving fecal blood testing,* **Gastroenterology** /1998), 114(1):227, dass Hämoglobin in Stuhl durch auf Papier nach Dehydratisierung stabil wäre, die quantitative Bestimmung aber für Anwender problematisch ist.

Es besteht somit ein Bedürfnis für eine präanalytische Methode, mit der eine fäkale Probe für eine proteinchemische Analytik aufbereitet und konserviert werden kann. Der Stand der Technik repräsentiert das Problem.

### KURZBESCHREIBUNG DER ERFINDUNG

Diese Anmeldung offenbart ein Verfahren nach Anspruch 1 und für das Verfahren geeignete Testbestecke und Reagenziensätze nach Anspruch 10. Bevorzugte Ausführungsformen und Aspekte der Erfindung sind den Unteransprüchen 2 bis 9 und 11-12 sowie den Beispielen zu entnehmen.

Das Verfahren zur präanalytischen Aufbereitung von fäkalen Proben (Fäzes, Kot, Kloake oder Stuhl) umfasst die Schritte: (a) Aufnehmen und Überführen einer bekannten Menge Probenmatrix in ein Gefäß mit einer bekannten Menge wässriger Proteinextraktionslösung; (b) Extrahieren des proteinösen Analyt aus der Matrix der Probe; (c) Auftragen eines bekannten Volumens wässrigen Extrakts auf ein Trägermaterial mit kapillaren und chromatographischen Laufeigenschaften, so dass sich der wässrige Extrakt mit den Zielproteinen auf dem Trägermaterial verbreitet; (d) Trocknen des Trägermaterials, wobei der Träger mit dem getrockneten Extrakt eine Konservierungs- und Transportform der fäkalen Probe darstellt. Zur anschließenden quantitativen Bestimmung der aminosäurenhaltigen bzw. des proteinösen Analyten im Stuhl: (e) Eluieren des Analyten von dem Trägermaterial in eine bekannte Menge Untersuchungspuffer; (f) Abtrennen des Trägermaterials vom Untersuchungspuffer; und (g) quantitatives Bestimmen des proteinösen Analyten durch eine enzymatische oder immunologische Reaktion. Die vorgenannte wässrige Probenextraktionslösung enthält im molaren Überschuss bezogen auf die Proteine und Aminosäuren, bevorzugt 1 bis 50 mM/L einer Ketocarbonsäure mit 1 bis 12 Kohlenstoffatomen, die beim Trocknen mit Aminosäuren und Proteinen im Extrakt wasserlösliche hydratisierte Salze bildet, sowie Puffersalze für einen pH zwischen 3 und 10, und fakultativ Detergenzien, Solubilisierungsmittel, Komplexierungsmittel, Redox-Mittel, Biozide, Adjuvanzien sowie Salze für die Einstellung der lonenstärke.

Die Ketocarbonsäuren können ausgewählt sein aus der Gruppe mit α-Ketomonocarbonsäuren, α-Ketodicarbonsäuren, β-Ketocarbonsäuren, β-Ketodicarbonsäuren, α-Ketopropionsäure (Brenztraubensäure), Acetessigsäure, Ketomalonsäure (Mesoxalsäure), Oxalessigsäure, Oxalbernsteinsäure, α-Ketoglutarsäure, Isocitrat, Oxohexansäure (Ketocapronsäure), Oxoheptansäure (Ketoönanthsäure), γ-Ketocarbonsäuren, Lävulinsäure, Phenylbrenztraubensäure, Ketoderivate und Ester der Terephthalsäure, sowie Mono- oder Diester der vorgenannten Ketocarbonäuren mit einem linearen oder verzweigten Alkohol mit 1 bis 6 Kohlenstoffatomen, Glyoxylsäure, Glyoxylsäureethylester, Brenztraubensäureethylester.

Der Extraktionspuffer kann enthalten: 0,001 bis 5,0 Gew.%, bezogen auf das Gesamtgewicht, vorzugsweise 0,005 bis 0,5 Gew.%, Zucker und/oder Aminozucker, bevorzugt ein konservierendes Mono-, Di- oder Trisaccharid, vorzugsweise Saccharose, Lactose, Maltose oder Trehalose. Der Aminozucker kann sein: Glucosamin, N-Methylglucosamin, Galactosamin oder Neuraminsäure. Zucker wie Sucrose oder Dextrose sind nicht ausgeschlossen. Besonders bevorzugt ist Trehalose wegen seines niederen Glasübergangspunkts und seiner Eigenschaften als "Wasserersatz" bei der Stabilisierung von Proteinen.

Bevorzugt kann der Probenextraktionspuffer ein oder mehrere Adjuvanzien enthalten, ausgewählt aus wasserlöslichem Farbstoff, pH-Indikator, Redox-Indikator, Fäulnisindikator, Methylenblau, Malachitgrün, Aromastoffe, Citronellal, Geraniol. Der Extraktionspuffer kann weiterhin ein Fließmittel enthalten, ausgewählt aus Polycarboxylat, Polycarboxylatether, Naphthalinsulfonat, Melaminsulfonat, Ligninsulfonat.

Eine bekannte Menge an Probenextrakt wird aufgetragen auf einen Träger, d.h., ein Flächengebilde mit guten Kapillar- und Verbreitungseigenschaften, so dass der Probenextrakt auf dem Flächengebilde bei Umgebungsbedingungen rasch eintrocknet. Ein vorteilhaftes Flächengebilde zum konservierenden Trocknen des Probenextrakts ist besonders ein Gebilde aus Fasern, die zu einem Vlies zusammengefügt sind, oder ein Träger, auf dem Fasern aufgetragen sind. Das Flächengebilde ist bevorzugt über zwei oder mehrere Verbindungselemente oder Stege mit einer größeren Fläche saugfähigen Materials verbunden. Hierdurch ist die Menge an aufgenommenen Probenextrakt definiert. Die Menge an trockenem Probenextrakt ergibt sich dann aus der Trocknungsfläche des Flächengebildes. Diese liegt bevorzugt zwischen 0,3 und 3,0 Quadratzentimeter, bevorzugt zwischen 0,5 und 2,0 cm², höchst bevorzugt bei ca. 0,78 cm² (Kreisfläche mit 1,0 cm Durchmesser).

Die fäkale Probe wird erfindungsgemäß extrahiert, je nach Analyten mit 10 bis 5000 Teilen Probenextraktionspuffer. Besonders günstig ist die Verwendung von 50 bis 250 Teilen Extraktionspuffer, um auch die Laufeigenschaften auf dem Flächengebilde zu vereinheitlichen. Da die Matrix der fäkalen Probe unverdauliche Fasern enthalten kann und weitere unverdaute Festbestandteile, kann der Extrakt vor dem Auftragen noch gefiltert werden, bspw. durch ein Siebfilter, oder die Festbestandteile können auch abzentrifugiert werden. Das Siebfilter kann in der Spitze des Probenaufnahmesystem angeordnet sein.

Ein anderer Aspekt der Offenbarung betrifft ein Testbesteck, umfassend ein Stuhlaufnahmesystem für die Aufnahme einer bekannten Menge fäkaler Probe in einen Probenextraktionspuffer, wie oben beschrieben, sowie ein Flächengebilde mit Kapillareigenschaften, wie oben beschrieben, für die Aufnahme, zum Trocken und für den Transport von trockenen Probenextrakt. Fläche und Fasermaterial sowie Probenextraktionspuffer sind bevorzugt ausgelegt für die Bestimmung von folgenden Parametern aus Stuhl bzw. Fäzes : Albumin, anti-Gliadin slgA, α1-Antitrypsin, Calprotectin, ß-Defensine (HBD-1, -2, -3, - 5, -6), Dopamin, anti-humane-Gewebetransglutaminase slgA, EDN (eosinophil-derived neurotoxin), Gallensäuren, Hämoglobin, Hämoglobin/Haptoglobin-Komplex, Histamin, H.pylori-Antigen, Ferritin, Lysozym, Laktoferrin, Myeloperoxidase, pankreatische Elastase, pankreatische Amylase, Präalbumin/Transthyretin, Serotonin, Zonulin.

Die Erfindung und ihre Vorteile werden nunmehr an Beispielen und Ausführungsformen mit Bezug auf die Abbildungen näher beschrieben. Diese dienen der Darstellung und Beschreibung. Die Offenbarung ist jedoch nicht hierauf beschränkt, sondern der beanspruchte Schutzumfang ergibt sich aus den anhängenden Ansprüchen.

### KURZ BESCHREIBUNG DER ABBILDUNGEN

Es zeigt:
- **Fig. 1**: ein Diagramm der Punktwolke und dem Ergebnis des Methodenvergleichs zwischen der Direktbestimmung aus Extraktflüssigkeit und der Bestimmung nach Lösen eines Trockenfäkalspots (DFS);

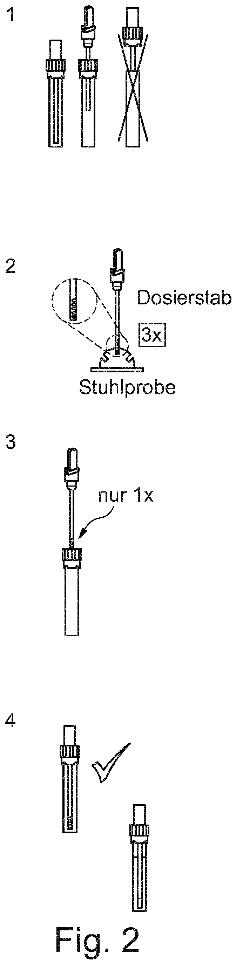

- **Fig. 2**: eine zeichnerische Darstellung des Stuhlaufnahmesystems zur Überführung einer definierten Menge an Stuhl (15 mg) in einen Extraktionspuffer (1,5 ml) gemäß der Erfindung;
- **Fig. 3**: einen photographischen Ausschnitt des Flächengebildes, wobei die Trocknungsfläche begrenzt ist durch Einstanzungen, und die über Stege fluidverbunden ist mit einem größeren Faservlies; die Trocknungsfläche kann für die Analyse herausgetrennt werden.

### EINGEHENDE BESCHREIBUNG DER ERFINDUNG

Die hier offenbarte Präanalytik für die quantitative Untersuchung fäkaler Proben umfasst das Aufnehmen und Überführen einer definierten Menge Probe in ein Gefäß mit Extraktionsmittel, ein Ablösen bzw. Extrahieren des zu quantifizierenden Analyten aus der Probenmatrix, beispielsweise durch Dispergieren der Probe in dem Extraktionspuffer; ein Abtrennen des Extrakts mit dem Analyten von festen Teilen der Untersuchungsmatrix, beispielsweise durch Passieren des Extrakts durch ein Siebfilter, Sedimentation oder Zentrifugation; ein Auftragen einer bekannten Menge Extrakt auf eine vorgegebene Fläche mit Kapillareigenschaften und chromatographischen Eigenschaften, so dass sich der Probenextrakt auf der Fläche verbreitet und bei üblichen Umgebungsbedingungen eintrocknen kann. Dies kann erfolgen durch Aufbringen einer bekannten Zahl von Tropfen auf die Trocknungsfläche. Trocknen des Probenextrakts auf der Fläche unter Umgebungsbedingungen, wonach der Träger mit dem getrockneten Probenextrakt eine stabile Lager- und Transportform darstellt. Die Trocknungsfläche ist bevorzugt in eine größere Fläche eingestanzt und mit dieser über einen oder mehrere Stege bzw. Halterungselemente verbunden. Überschüssige Flüssigkeit kann so auf die Umgebungsfläche abfließen bzw. wird von dieser abgesaugt. Die vorgegebene Trocknungsfläche kann dann für die Analytik aus der Umgebung herausgebrochen werden. Dies vereinheitlicht die Menge an aufgetragener Probenflüssigkeit. Sie ergibt sich im Übrigen aus der Viskosität und Fluidität des Extraktionspuffers auf der Trocknungsfläche. Viskosität und Fluidität des Extrakts können eingestellt werden über die Zuckerkonzentration im Extraktionspuffer. Die Trocknungsfläche mit dem Trägermaterial ist bevorzugt an einem Briefchen oder Umschlag befestigt. Dieses kann vorbeschriftet oder für eine Beschriftung vorbereitet sein. Nach Auftragen und Trocknen des Extraktionspuffers auf der Trocknungsfläche ist die Präanalytik abgeschlossen.

Die Präanalytik zeichnet sich dadurch aus, dass das Extraktionsmittel eine Ketocarbonsäure enthält, die mit Aminosäuren und Proteinen koordinierte Salze bildet und einen Ersatz für die koordinierten Wasserstoffbrückenbindungen stellen kann. Es wurde gefunden, dass Proteine und aminosäureartige Verbindungen in Gegenwart von Ketocarbonsäuren auch nach einem Trocknen auf einem Träger bzw. einer Trocknungsfläche nicht denaturieren sind oder über hydrophobe Wechselwirkungen auf der Oberfläche gebunden, sondern in so einem Extrakt auch nach dem Trocknen quantitativ löslich bleiben.

Das offenbarte Verfahren umfasst nach der präanalytischen Aufbereitung von fäkalen Proben (Fäzes, Kot, Kloake oder Stuhl) auch die Schritte zur quantitativen Bestimmung des Analyten, nämlich ein Eluieren des Analyten von der Trocknungsfläche in eine bekannte Menge Untersuchungspuffer; ein Abtrennen des Trägermaterials vom Untersuchungspuffer; und ein quantitatives Bestimmen des Analyten durch eine enzymatische oder immunologische Reaktion.

Die wässrige Extraktionslösung für die fäkale Probe enthält im molaren Überschuss, bezogen auf die extrahierten Proteine und Analyten, bevorzugt 1 bis 50 mM/L Ketocarbonsäure mit 1 bis 12 Kohlenstoffatomen, die mit aminosäurehaltigen Verbindungen wasserlösliche koordinierte hydratisierte Salze bilden, sowie Puffersalze für einen pH zwischen 3 und 10. Detergenzien, Solubilisierungsmittel, Komplexierungsmittel, Redox-Mittel, Biozide, Adjuvanzien sowie Salze für die Einstellung der lonenstärke sind fakultativ zugegen.

Die Ketocarbonsäuren sind bevorzugt ausgewählt aus der Gruppe mit α-Ketomonocarbonsäuren, α-Ketodicarbonsäuren, β-Ketocarbonsäuren, β-Ketodicarbonsäuren, α-Ketopropionsäure (Brenztraubensäure), Acetessigsäure, Ketomalonsäure (Mesoxalsäure), Oxalessigsäure, Oxalbernsteinsäure, α-Ketoglutarsäure, Isocitrat, Oxohexansäure (Ketocapronsäure), Oxoheptansäure (Ketoönanthsäure), γ-Ketocarbonsäuren, Lävulinsäure, Phenylbrenztraubensäure, Ketoderivate und Ester der Terephthalsäure, sowie Mono- oder Diester der vorgenannten Ketocarbonäuren mit einem linearen oder verzweigten Alkohol mit 1 bis 6 Kohlenstoffatomen, Glyoxylsäure, Glyoxylsäureethylester, Brenztraubensäureethylester.

Der Extraktionspuffer kann zudem ein oder mehrere konservierende Zucker enthalten, die beim Eintrocknen des Puffers mit Proteinen hydratartige Salze bilden, bei Raumtemperatur auf dem Träger nicht kristallisieren, sondern glasartig werden. Der Extraktionspuffer kann Mono, Di- oder Trisaccharide enthalten wie Saccharose, Lactose, Maltose oder Trehalose und als Trisaccharid Raffinose. Bevorzugt sind Saccharose, Lactose, Maltose oder ganz besonders Trehalose. Auch können Aminozucker enthalten sein, d. h. Monosaccharide, die anstelle einer Hydroxygruppe eine primäre, sekundäre oder tertiäre Amino- oder eine acylierte Aminogruppe (-NH-CO-R) besitzen. Bevorzugt sind Glucosamin, N-Methylglucosamin, Galactosamin und Neuraminsäure. Der Zucker/Aminozucker liegt in der Zubereitung für den Extraktionspuffer bevorzugt in einer Menge vor, dass nach Trocknen und erneutem Eluieren von dem Träger im vorgesehenen Volumen an Untersuchungspuffer er in der Lösung in einer Konzentration von circa 1 bis 200 mg/ml vorliegt. Bevorzugt liegt der Zucker im Untersuchungspuffer in einer Konzentration von 30 bis 50 mg/ml vor. Der Extraktionspuffer kann somit enthalten 0,001 bis 5,0 Gew.%, bezogen auf das Gesamtgewicht, vorzugsweise 0,005 bis 0,5 Gew.% Zucker und/oder Aminozucker. Besonders bevorzugt ist Trehalose. Trehalose ist zwar schwach hygroskopisch, sie besitzt aber einen vergleichsweise hohen Gelier- und Glasübergangspunkt. Der Glasübergang beschreibt den Temperaturbereich, in dem eine amorphe Substanz vom festen glasartigen Bereich in den viskoelastischen, "gummiartigen" Bereich übergeht. Dieser Bereich spielt eine essentielle Rolle bei der Verkapselung von Substanzen in amorphen kohlenhydrathaltigen Matrices wie Cellulosefasern. Trehalose schützt in der Natur Zellen vor Verletzungen durch Eiskristalle bei Frost oder Tiefkühlprozessen und auch bei Trockenheit. In gewisser Weise ist Trehalose funktionell gleichartig zu Saccharose, besitzt aber andere Glaspunkt- und Stabilisierungseigenschaften. Trehalose kommt natürlich in Pflanzen und Pilzen und in der Hämolymphe vieler Insekten vor. Trehalose ist chemisch, thermisch und gegenüber Säure stabil und in Wasser rasch löslich, wobei bei niedrigen Temperaturen Trehalose weniger und bei höheren Temperaturen stärker löslich ist als Saccharose. Im Gegensatz zu den Disacchariden ist Trehalose aber nicht hydrolysierbar und sie kann nicht an einer Maillard-Reaktion mit den Aminosäuren oder Proteinen teilnehmen. Günstig ist auch, dass sie eine hohe Klebkraft auf vielen Kunststoffen und auf Cellulose besitzt und dadurch verhindert, dass der getrocknete Probenextrakt von der Oberfläche des Trägers während des Transports abbröselt.

Der Extraktionspuffer kann ein oder mehrere Adjuvanzien enthalten, ausgewählt aus wasserlöslichem Farbstoff, pH-Indikator, Redox-Indikator, Fäulnisindikator, Methylenblau, Malachitgrün, Aromastoffe, Citronellal, Geraniol. Der Extraktionspuffer kann weiterhin ein Fließmittel enthalten, ausgewählt aus Polycarboxylat, Polycarboxylatether, Naphthalinsulfonat, Melaminsulfonat, Ligninsulfonat.

Die fäkale Probe wird extrahiert, je nach Analyten mit 10 bis 5000 Teilen Probenextraktionspuffer. Besonders günstig ist die Verwendung von 50 bis 250 Teilen Extraktionspuffer, um auch die Laufeigenschaften auf dem Flächengebilde zu vereinheitlichen. Da die Matrix der fäkalen Probe unverdauliche Fasern enthalten kann und weitere unverdaute Festbestandteile, kann der Extrakt vor dem Auftragen noch gefiltert werden, bspw. durch ein Siebfilter, oder die Festbestandteile können auch abzentrifugiert werden. Das Siebfilter kann in der Spitze des Probenaufnahmesystem angeordnet sein.

Der Probenextraktionspuffer wird in bekannter Menge aufgetragen auf einen Träger, d.h., ein Flächengebilde mit guten Kapillar- und Verbreitungseigenschaften, so dass er auf dem Flächengebilde bei Umgebungsbedingungen rasch eintrocknet. Ein vorteilhaftes Flächengebilde zum konservierenden Trocknen des Probenextrakts ist besonders ein Gebilde aus Fasern, die zu einem Vlies zusammengefügt sind oder ein Träger, auf dem Fasern aufgetragen sind. Das Flächengebilde ist bevorzugt über zwei oder mehrere Verbindungselemente oder Stege mit einer größeren Fläche saugfähigen Materials verbunden. Hierdurch ist die Menge an aufgenommenen Probenextrakt definiert. Die Menge an trockenem Probenextrakt ergibt sich dann aus der Trocknungsfläche des Flächengebildes. Diese liegt bevorzugt zwischen 0,3 und 3,0 Quadratzentimeter, bevorzugt zwischen 0,5 und 2,0 cm², höchst bevorzugt bei ca. 0,78 cm² (Kreisfläche mit 1,0 cm Durchmesser).

Ein Aspekt der Offenbarung betrifft ein abgestimmtes Testbesteck für den Patienten bzw. die Testperson, umfassend ein Stuhlaufnahmesystem für die Aufnahme einer vorbestimmten Menge fäkaler Probe in eine vorgegebene Menge Probenextraktionspuffer, wie oben beschrieben, sowie ein Flächengebilde mi t Kapillareigenschaften, wie oben beschrieben, für die Aufnahme, zum Trocken und für den Transport von trockenen Probenextrakt. Das Stuhlaufnahmesystem ist so ausgelegt, dass die Testperson selbst eine bekannte Anzahl definierter Tropfen Probenextrakt - entsprechend beispielsweise 200 µl - auf die vorgestanzte Trocknungsfläche auftrocknen kann. Die Mengen ( Fläche und Fasermaterial sowie Probenextrakt) sind bevorzugt ausgelegt für die Bestimmung von folgenden Parametern aus Stuhl bzw. Fäzes: Albumin, anti-Gliadin slgA, α1-Antitrypsin, Calprotectin, ß-Defensine (HBD-1, -2, -3, -5, -6), Dopamin, anti-humane-Gewebetransglutaminase slgA, EDN (eosinophil-derived neurotoxin), Gallensäuren, Hämogloblin, Hämoglobin/Haptoglobin-Komplex, Histamin, H.pylori-Antigen, Ferritin, Lysozym, Laktoferrin, Myeloperoxidase, pankreatische Elastase, pankreatische Amylase, Präalbumin/Transthyretin, Serotonin, Zonulin.

Bevorzugt erfolgt die quantitative Bestimmung des Analyten nach Ablösen vom Trägermaterial im Untersuchungspuffer über eine immunologische Reaktion. Der Bindungsassay kann beispielsweise erfolgen in einem ELISA (enzyme-linked immuno-sorbens assay), RIA (radioimmunoassay), FIA (fluorescence immunoassay), LIA (luminescence immunoassay), oder ILMA (immuno luminometric assay). Besonders bevorzugt sind nichtradioaktive Nachweissysteme auf der Basis von Fluoreszenz und Chemilumineszenz-Markern (FIA oder LIA).

Wesentlich für die Präanalytik ist das Auftragen des Probenextrakts auf eine definierten Trocknungsfläche, d.h. einen Träger mit Kapillarwirkung. Weiterhin ist essentiell, dass der Analyt bzw. das Zielprotein auf der Trocknungsfläche nicht denaturiert und irreversibel gebunden wird, denn nach der zwischenzeitlichen Konservierung auf der Trocknungsfläche muss der Analyt wieder quantitativ in Lösung gehen. Bei der Extraktion der Probenmatrix wird der Analyt bereits erstmals in Lösung gebracht, der Extrakt durch Sedimentation, Floating, Filtration oder Zentrifugation von den Festanteilen der Matrix abgetrennt, und dann eine Menge Extrakt auf die Matrix der Trocknungsfläche, bzw. auf den Träger aufgetragen, z.B. in Form von einer vorgegebenen Zahl von Tropfen. Nach Auftragen auf die Trocknungsfläche mit den Kapillareigenschaften erfährt das Proteingemisch des Extrakts aber eine partielle chromatographische Auftrennung, wobei der Analyt auch von den Verdauungsenzymen separiert wird. Die im Extrakt vorhandenen Darmbakterien werden in einer glasartigen Zuckerschicht inaktiviert. Der Analyt, das Zielprotein endet trocken und geschützt als lösliches Salz der beta-Ketocarbonsäure auf der Oberfläche und in den Hohlräumen des Trägermaterials.

Das Verfahren verlangt die Verwendung eines Flächengebildes mit guten Kapillar- und Verbreitungseigenschaften. Dies ist gegeben bei einem Vliesmaterial, einem Flächengebilde aus Fasern, die zu einem Vlies zusammengefügt sind oder einem Träger, auf dem Fasern aufgetragen sind. Gerichtete Fasern oder geordnete Filamente haben hohe

Kapillarwirkung und erlauben ein Verbreiten und Trocknen der Probenextraktionslösung bei Umgebungsbedingungen. Die Fasern können ausgewählt sein aus Cellulosefaser, Glasfaser, Fasern aus Polyester, Polyethersulfon (PES), Polyamid (Nylon), Polypropylen, Polytetrafluorethylen (PTFE), Polycarbonat, Cellulosenitrat oder Celluloseacetat, sowie Mischungen dieser Fasern. Das Vliesmaterial kann wegen der unterschiedlichen Verfestigungsverfahren auch Papieren, Folien oder faserverstärkten Kunststoffen gleichen. Andere Flächengebilde mit guten Kapillar- und Verbreitungseigenschaften sind chromatographische Dünnschichtplatten, also einer Folie oder Platte mit einer dünnen Schicht eines stationären Trennmaterials wie Kieselgur, die dann von der wässrigen Probenextraktionslösung durchwandert wird.

Das Flächengebilde ist vorteilhaft ausgelegt für eine Aufnahme und zum Auftrocknen von 10 bis 1000 µl wässrigen Probenextrakts, bevorzugt 50 bis 500 µl Die Trocknungsfläche kann so ausgelegt sein, dass ein Zuviel an Extraktionslösung an zwei oder mehreren Stellen über Verbindungselemente, Stege oder Zähne, abgeleitet wird. Die Trocknungsfläche kann Teil eines größeren Flächengebilde sein und umgeben von Schlitz- und Lochstanzungen, so dass sie aus der größeren Fläche herausgebrochen werden kann. Es sind viele Perforationsmuster und -strukturen zur Begrenzung der Trocknungsfläche möglich. In einer Ausführungsform sind ein oder mehrere Trocknungsflächen von der Größe einer Münze (Euro- oder Dollarstück) oder einer Briefmarke in einer größeren Fläche mit Vliesmaterial eingestanzt. Mehrere definierte Trocknungsflächen erlauben mehrere quantitative Bestimmungen von Stuhlparametern.

Der Träger mit dem getrockneten Probenextrakt wird vor der Analyse bzw. dem Ablösen der Stuhlproteine dann aus der größeren Fläche herausgebrochen. Hierdurch kann die Auftragsmenge und die Menge an getrockneten Probenextrakt genau begrenzt und standardisiert werden. Die Dicke des saugfähigen Flächengebildes ist so ausgelegt, dass auf dem Münz- oder Briefmarken-großen Träger ca. 200 bis 250 µl Probenextrakt festgehalten kann. Der trockene Probenextrakt auf dem Träger bzw. Vlies oder Fasermaterial repräsentiert die über mehrere Tage und Wochen stabile Lager- und Transportform und sie kann mit der Post und ohne große Logistikzwänge an das untersuchende klinische Labor gesandt werden kann. Ist der Trocknungsträger in einem Umschlag oder einer Manschette angeordnet, sind auch Probleme mit der Hygiene gelöst. Geruch ist vor allem bei flüssigen Transport- und Stabilisierungszuständen ein Problem.

Es werden nun Beispiele der Präanalytik und der Bestimmung beschrieben. Die Offenbarung ist jedoch nicht auf die Beispiele beschränkt, sondern der Schutzumfang ergibt sich aus den Ansprüchen.

### BEISPIELE

### Example 1 - Quantitative Bestimmung von pankreatischer Elastase im Stuhl

*Allgemeines*: Die pankreatische Elastase hat ein Molekulargewicht von 26 kDa und ist eine Serinprotease. Sie wird zusammen mit anderen Verdauungsenzymen von den Azinuszellen der Bauchspeicheldrüse als Proenzyme sezerniert und dann im Zwölffingerdarm durch Spaltung aktiviert. Während der Darmpassage ist sie an Gallensalze gebunden und ist so vor einem Verdau geschützt. Die Konzentration von pankreatischer Elastase im Stuhl ist ein Maß für die sekretorische Leistungsfähigkeit der Bauchspeicheldrüse. Die Konzentrationsbestimmung im Stuhl erlaubt die Diagnose einer exokrinen Pankreasinsuffizienz und sie ist angezeigt bei unklaren Durchfällen, Verstopfung, Fettstühlen, Blähungen, Gewichtsverlust, Oberbauchschmerzen sowie Nahrungsmittelunverträglichkeiten. Die Bestimmung im Stuhl erlaubt auch eine Überwachung der exokrinen Pankreasfunktion bei Mukoviszidose, Diabetes mellitus oder chronischer Bauchspeicheldrüsenentzündung. Eine regelmäßige Bestimmung sollte in allen diesen Fällen erfolgen.

*Pränalytisches Testbesteck:* Für die Präanalytik wurde an die Patienten ausgehändigt ein Stuhlaufbereitungssystem (Immundiagnostik AG, Bensheim, DE - Artikel-Nr. K 6998SAS), wobei das Stuhlröhrchen 1,5 ml erfindungsgemäßen Stuhlextraktionspuffer enthielt (20mM Na₂HPO₄, 2,6 mM KH₂PO₄, 2,7 mM KCl, 137 mM NaCl, 50 mM β-Natriumglutarat, 20 mM Trehalose, 0,1% SDS, 1% Triclosan^{®}, Methylenblau, Citronellal). Die mit der Stuhlaufnahmelanze aufnehmbare Stuhlmenge war über die Größe der Einkerbungen und Dellen auf 15 Milligramm ausgelegt; der Verdünnungsfaktor betrug somit 1:100. Der Rohstuhl wurde mit der Lanze in den Extraktionspuffer überführt. Ein Überschuss an Stuhl wird bei dem ausgehändigten System beim Überführen zwangsläufig abgestreift. Der Stuhl sollte dann vom Patienten im Extraktionspuffer homogen dispergiert werden und nach 10 Minuten sollte er an "homogener Stelle" 200 Mikroliter (4 Tropfen) mit einer kalibrierten Minipipette (Minivette^{®} von Sarstedt, DE) entnehmen und auf ein rundes Cellulosevlies (Whatman, US) von 1 cm Durchmesser tropfen. Auf dieser Trocknungsfläche verbreitet sich der wässrige Stuhlextrakt zwangsläufig und trocknet bei Raumtemperatur bzw. Umgebungsbedingungen rasch ein. Die Trocknungsfläche war in einer größere Fläche Cellulosevlies eingestanzt, so dass ein eventueller oder vom Patienten beabsichtiger Überschuss an flüssigem Extrakt ("der 5. Tropfen") abgeleitet wurde. Die Trocknungsfläche repräsentierte eine stabile Transport- und Lagerform der Untersuchungsprobe, die an das klinische Labor zurückgesandt wird.

Vergleichsreihen zeigten, dass die Lagerung der Probe auf dem Faservlies über vier Wochen Tage insoweit stabil ist, als die Lagerzeit keinen substantiellen Einfluss auf das quantitative Ergebnis hatte. Das Faservlies konnte im Umschlag auf postalischem Weg an das Labor gesandt werden. Normalerweise beträgt die Stabilität von pankreatischer Elastase im Rohstuhl gekühlt (4-8°C) oder bei Raumtemperatur knapp 3 Tage und nur bei -20°C bis zu einem Jahr (Stein, J. et al., Immunoreactive elastase I: clinical evaluation ofa new noninvasive test of pancreatic function. Clinical Chemistry, 1996, 42(2):222-6; Nandhakumar, N. & Green, M.R., Interpretations: How to use faecal elastase testing. Archives of disease in childhood. Education and Practice Edition, 2010, 95(4):119-23]. Das Einfrieren hat den Nachteil, dass es mit einem Einfrier-/Auftauzyklus verbunden ist. Das Trocknungsverfahren hat den Vorteil, dass mehrere Proben aus einem Stuhl hergestellt und getrennt abgearbeitet werden können, dass man also verschiedene Parameter und Marker zugleich untersuchen kann. Zu beachten ist nur der Verdünnungsfaktor für den jeweiligen Parameter.

*Quantitative Bestimmung.* Die Bestimmung der pankreatischen Elastase auf dem Faservlies erfolgte mit einem herkömmlichen Elastase-ELISA (Immundiagnostik AG, Bensheim, DE - Artikel-Nr. K 6915). Die Trocknungsfläche mit dem Fäkalextrakt wurde aus dem Vliesmaterial herausgetrennt und alle Salze darauf in 1,0 ml ELISA-Waschpuffer gelöst, so dass ein "zweiter Extrakt" resultierte. Die vom Faservlies abgelöste pankreatische Elastase wurde gebunden von monoklonalen Anti-Elastase-Antiköpern, fixiert auf einer Mikrotiterplatte. Nichtgebundene Proteine des zweiten Extrakts wurde in Waschschritten von der Mikrotiterplatte abgespült und die gebundene pankreatische Elastase detektiert mit Peroxidase-markierten zweiten Antikörpern (Maus-anti-humane-Pankreaselastase), dem sogenannten Antikörper-Konjugat. Nach weiteren Waschschritten wurde eine Lösung mit Tetramethylbenzidin (TMB) als Substrat der Peroxidase für eine Farbreaktion zugesetzt. Die Farbentwicklung auf der Mikrotiterplatte ist dann proportional zur Menge an Analyt in der Probe bzw. im Vergleichsstandard. Über eine parallele Standardkurve kann dann mittels Vergleichs die Konzentration in der Probe bestimmt werden. Unabhängig von der Ausgangsprobe und der Trocknungszeit auf dem Faservlies geht mit dem beschriebenen Stuhlextraktionspuffer die pankreatische Elastase reproduzierbar und quantitativ wieder in Lösung und kann so auch nach Transport auf dem Faservlies bestimmt werden.

Konkret wurde 100 µl Standard, Kontrolle bzw "Zweitextrakt" (1 ml Waschpuffer) in Doppelwerten auf eine Antikörper-beschichtete Mikrotiterplatte pipettiert und 30 Minuten unter Schütteln bei Raumtemperatur inkubiert. Die Mikrotiterplatte wurde 5x mit Waschpuffer gewaschen und in jede Kavität 100 µl Antikörper-Konjugat pipettiert. Es folgten 30 Minuten Inkubation unter Schütteln bei Raumtemperatur. Die Mikrotiterplatte wurde mehrfach mit Waschpuffer gewaschen, und in jede Kavität 100 µl TMP-Substrat pipettiert und 15 Minuten lichtgeschützt bei Raumtemperatur inkubiert. Die Farbreaktion wurde gestoppt durch Zugabe einer Stopplösung (100 µl H₂SO₄-Lösung) in jede Kavität. Die Messung erfolgte dann im Photometer bei einer Wellenlänge von 450 nm.

### Example 2 - Methodenvergleich Direktbestimmung und Trockenfäkalspot (DFS)

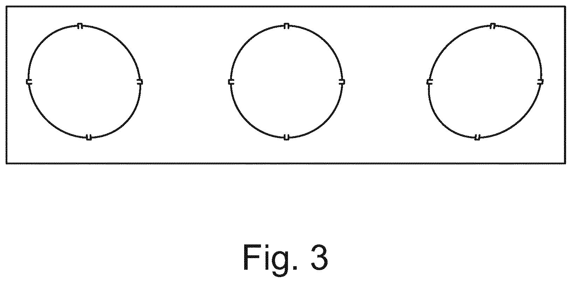

Es wurden fäkale Rohproben (gelagert bei -20°C) auf die Konzentration von pankreatischer Elastase direkt und nach Lösen eines Trockenfäkalspots von einem Faservlies untersucht. Für die Direktbestimmung wurde 15 mg Stuhlprobe mit dem im Beispiel 1 beschriebenen Stuhlaufnahmesystem (SAS), in 1,5 ml Extraktionspuffer (Verdünnung 1:100) überführt, dispergiert und die Suspension 10 Minuten bei Raumtemperatur equilibriert; siehe Figur 3. Der erste Extrakt (Verdünnung 1:100) wurde 1:100 mit ELISA-Waschpuffer weiterverdünnt, so dass die Endverdünnung 1:10.000 betrug. Die immunologische Bestimmung der pankreatischen Elastase erfolgte mit 100 µl Endverdünnung pro Kavität wie in Beispiel 1.

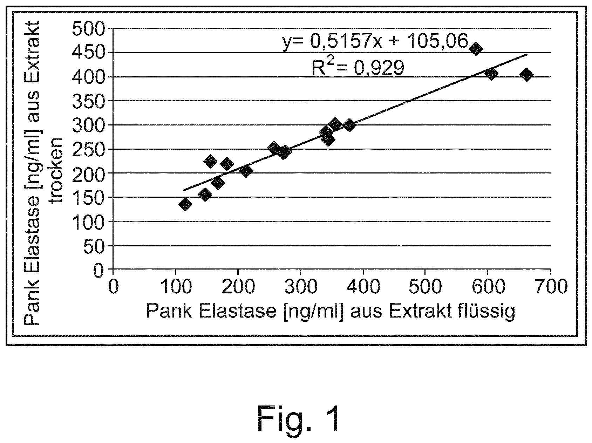

Für die Bestimmung nach Ablösen des Trockenfäkalspots (DFS - dried fecal spot) von dem Faservlies wurde 100 µl Extraktionspuffer (Verdünnung 1:100) auf Faservlies getropft, bei Raumtemperatur eingetrocknet und 7 Tage (eine Woche) bei Umgebungstemperatur in einem Briefumschlag im Regal aufbewahrt. Danach wurden die Trocknungsflächen aus dem Vliesmaterial herausgetrennt und jeweils 10 Minuten in einem 1,5 ml Eppendorf-Röhrchen mit 1,0 ml Waschpuffer (Verdünnung 1:1000) unter Vortexen behandelt. Das Faservlies wurde abzentrifugiert und 100µl Überstand mit 900µl ELISA-Waschpuffer verdünnt (Verdünnung 1:10.000). Es wurde 100µl dieser Endverdünnung im Test pro Vertiefung eingesetzt. Die Ergebnisse des Methodenvergleichs sind Tabelle 1 zu entnehmen und in Figur 1 als Punktewolke graphisch dargestellt.

**TABELLE 1**

| *Methodenvergleich pankreatische Elastase in Stuhl bei direkter Bestimmung und nach Trocknen und Ablösen von einem Faservlies* | | |
|---|---|---|
| Probe Nr. | Bestimmung direkt in flüssigem Extrakt | Bestimmung nach Ablösen des DFS von Faservlies |
| Pankreas-Elastase Konzentration in Stuhl | [µg / ml] | [µg / ml] |
| 1 | 213.6 | 204 |
| 2 | 147,5 | 156,45 |
| 3 | 167,8 | 180,3 |
| 4 | 605,5 | 406,25 |
| 5 | 155,4 | 225,55 |
| 6 | 582,2 | 458,25 |
| 7 | 340,9 | 285,05 |
| 8 | 275,4 | 244,15 |
| 9 | 662,1 | 405,55 |
| 10 | 343,2 | 270,3 |
| 11 | 356,3 | 302,1 |
| 12 | 115,5 | 136,35 |
| 13 | 182,2 | 218,6 |
| 14 | 258,4 | 251,55 |
| 15 | 271,7 | 243,75 |
| 16 | 377,5 | 299,95 |

Die Korrelation zwischen diesen beiden Messverfahren war sehr stark (R = 0,96). Das Bestimmtheitsmaß R²= 0,929; Figur 1 zeigt die Punkteanalyse (Scatterplot). Die Richtigkeit bzw. Korrelation ist hier ein Maß für die Abweichung eines Messwertes vom "wahren" Wert aufgrund eines systematischen Fehlers. Belegt wurde sie hier über den Vergleich mit einem validierten Verfahren, der Direktbestimmung in "Flüssigkeit". Die Methoden waren somit absolut gleichwertig.

### Example 3 - Vergleich der Präzision der Methoden

Die nachstehenden Angaben zur Präzision (Intra-Assay) geben an, wie stark die Analysenwerte bei wiederholter Analyse streuen. Die Inter-Assay-Unpräzision bzw. die Reproduzierbarkeit wurde gleichfalls bestimmt. Tabelle 2 zeigt die Ergebnisse zur Reproduzierbarkeit bzw. zur Streuung der Ergebnisse für zwei Proben ("11" - mittlerer Wert - und "22" - hoher Wert) an 20 Wiederholungen.

**Tabelle 2Streuung (n = 20) der Messwerte bei Bestimmungen aus einem Trockenfäkalspot**

| Probe Nr. "11" | Probe Nr. "22" |
|---|---|
| | |
| 321.3 | 537,4 |
| 307,9 | 555 |
| 268,0 | 548,2 |
| 304.3 | 480,7 |
| 302,6 | 540.0 |
| 291,4 | 528,8 |
| 281,1 | 518.0 |
| 276,4 | 521,4 |
| 286,5 | 530,9 |
| 281,9 | 552,7 |
| 285,5 | 539,8 |
| 326,2 | 562.0 |
| 340,8 | 491,7 |
| 298,8 | 509,6 |
| 282,2 | 544,0 |
| 302,7 | 530,2 |
| 289,8 | 525,8 |
| 289,4 | 494,8 |
| 298,3 | 501,1 |
| 297,9 | 512.8 |

Die Standardabweichung (n=20) betrug bei der Rohprobe "11" mit mittelhoher Pankreas-Elastase-Konzentration (MW = [296,65 µg/ml]) gleich 17,25 µg/mL. Der prozentuale Variationskoeffizient war 5,81 %. Bei einer Rohprobe mit hoher Pankreas-Elastase-Konzentration (MW= 526,25 [µg/ml]) betrug die Standardabweichung 21,89.

Die Inter-Assay-Unpräzision bzw. die Reproduzierbarkeit der Bestimmung der Pankreas-Elastase-Konzentration im Stuhl an verschiedenen Tagen wurde bestimmt für Bestimmungen aus Trockenfäkalspot (DFS) und Direktbestimmung aus Stuhlextraktionsflüssigkeit.

**TABELLE 3**

| *Unpräzision der Methoden bei einer Bestimmung* ***(n* =** ***20**) Stuhlextrationsflüssigkeit (SES) und Trockenfäkalspot (DFS)* aus | | | |
|---|---|---|---|
| | Probe "14" | Probe "14" | Probe "15" |
| Datum | SES | DFS | SAS |
| | | | |
| 161031 1 | 206 | 201 | 304 |
| | 192 | 205 | 314 |
| 161031 2 | 166 | 213 | 317 |
| | 166 | 215 | 321 |
| 161101 1 | 234 | 215 | 312 |
| | 230 | 202 | 300 |
| 161101 2 | 216 | 245 | 342 |
| | 226 | 244 | 320 |
| 161102 1 | 217 | 231 | 306 |
| | 208 | 227 | 306 |
| 161102 2 | 241 | 211 | 325 |
| | 234 | 216 | 307 |
| 161104-1 | 254 | 295 | 339 |
| | 248 | 300 | 368 |
| 161104 2 | 206 | 208 | 342 |
| | 256 | 197 | 365 |
| 161110 1 | 238 | 232 | 364 |
| | 243 | 230 | 363 |
| 161110 2 | 257 | 231 | 366 |
| | 254 | 257 | 340 |
| | | | |
| ***Anzahl*** | ***20*** | ***20*** | ***20*** |
| ***MW (µa*/*mL)*** | ***224.60*** | ***228,75*** | ***331,05*** |
| ***STABWN s*** | ***26,77*** | ***27.73*** | ***23,31*** |
| ***VK [%]*** | ***11.92*%** | ***12.12%*** | ***7.04%*** |

Die Ergebnisse zeigen, dass Präzision und Reproduzierbarkeit von der Trocknung des Stuhlextrakts auf einem Faservlies nicht substantiell beeinflusst werden. Damit stellt das präanalytische Verfahren einen hohen praktischen Wert bereit, denn es behebt die Probleme mit dem Transport und der Stabilität von Stuhlproben.

### Example 4 - Bestimmung von Calprotectin aus Trockenstuhlspots (DFS)

Fäkales Calprotectin ist ein Marker mit hoher negativer prädiktiver Aussagekraft in Bezug auf gastrointestinale Erkrankungen. Ist der Calprotectin-Spiegel im Stuhl niedrig, liegt mit hoher Wahrscheinlichkeit keine organische Erkrankung des Intestinaltrakts vor. Es ist daher bedeutsam für die Unterscheidung zwischen Patienten mit Colon irritabel und solchen mit chronisch-entzündlichen Darmerkrankungen (CED). Der Nachweis aus dem Stuhl mittels ELISA oder turbidimetrischer aus einem flüssigen Stuhlextrakt korreliert gut mit histologischen und endoskopischen Befunden der Krankheitsaktivität bei Morbus Crohn und Colitis ulcerosa, sowie mit der Standardreferenz für die CED-Aktivität, der Messung der fäkalen Exkretion 111-Indium-markierter neutrophiler Granulozyten. Der Granulozytentest ist aber sehr aufwändig (Krankenhausaufenthalt, Isotopenbestimmung und Entsorgung) und belastend. Calprotectin im Stuhl ist ein idealer Verlaufsmarker bei M. Crohn oder nach Polypenabtragung. Erste Versuche haben gezeigt, dass aus Trockenfäkalspots auch die Konzentration von Calprotectin im Stuhl immunologisch bestimmt werden kann. Die Bestimmung von Calprotectin erfolgte analog Beispiel 1. Der Methodenvergleich (DFS zur Nassanalyse) ergab eine hohe Messwertekorrelation (R= 0,95); und ein adjustiertes Bestimmtheitsmaß R² = 0,918.

### Example 5 - Bestimmung von okkultem Blut aus Trockenstuhlspots (DFS)

Der Nachweis von Hämoglobin in Stuhl kann als Marker für gastrointestinale Blutungen für die Darmkrebsvorsorge dienen. Blut in Stuhl kann von Tumoren oder Polypen im Darm herrühren. Andere mögliche Indikationen sind, Morbus Crohn, Colitis Ulcerosa, etc. Wenn Hämoglobin im Stuhl nachweisbar ist, muss durch eine Darmspiegelung abgeklärt werden, woher die Blutung stammt bzw. ob ein Tumor oder eine Vorstufe im Darm vorhanden ist. In Kombination mit der Darmspiegelung kann der Fäzes-Immuntest auf Hb nachweislich das Risiko verringern, an Darmkrebs zu sterben. Die Probennahme und quantitative Bestimmung können analog Beispiel 1 erfolgen. Als besonderer Vorteil ergibt sich dabei eine hohe Probenstabilität bei Raumtemperatur. Pro Tag wird Hämoglobin im Stuhl bei Raumtemperatur bis zu 50% abgebaut, so dass präanalytisch Rohstuhl eingefroren werden muss. Selbst in einem Stabilisierungspuffer beträgt der Abbau von okkultem Hämoglobin bei Raumtemperatur noch ca. 3 bis 7% pro Tag, oft sogar mehr. Wird hingegen Stuhl wie in Beispiel 1 als Trockenstuhlspot auf einem Vliespapier konserviert, ist das Hämoglobin über Wochen quantitativ stabil und die Probe kann mit der Post in ein Labor zur Analyse versandt werden. Da geringe Mengen Blut regelmäßig mit dem Stuhl ausgeschieden werden, muss die Bestimmung auf okkultes Hämoglobin quantitativ erfolgen, nicht nur analytisch. Da die Stuhlprobe in Bezug auf okkultes Blut im Stuhl stabil ist, kann ein Schwellenwert ermittelt, ab wann eine Darmspiegelung indiziert ist. Dieser Schwellenwert ist wie bei anderen Tests in diesem Bereich auch auf die gesuchte Sensitivität und Spezifität zu optimieren (siehe Gies A et al, Direct Comparison of Diagnostic Performance of 9 Quantitative Fecal Immunochemical Tests for Colorectal Cancer Screening, Gastroenterology, 2018, 154:93-104)

## Patentansprüche

1. Verfahren zur präanalytischen Aufbereitung einer Probe mit Fäzes, Kot oder Stuhl für eine quantitative Bestimmung eines proteinösen Analyten, umfassend die Schritte:
(a) Aufnehmen der Probe und Überführen einer bekannten Menge in ein vorbereitetes Gefäß mit einer bekannten Menge wässriger Extraktionslösung;
(b) Dispergieren und Extrahieren des proteinösen Analyt aus der Probenmatrix;
(c) Abtrennen des wässrigen Extrakts von den festen Matrixbestandteilen und Auftragen eines bekannten Volumens wässrigen Extrakts auf ein trockenes Trägermaterial, das Kapillareigenschaften und chromatographische Eigenschaften besitzt, so dass der Extrakt sich auf dem Trägermaterial verläuft und verbreitet;
(d) Trocknen des Trägermaterials bei Umgebungstemperatur, wobei der Träger mit dem trockenen Extrakt eine Konservierungs- und Transportform darstellt;
(e) Eluieren des proteinösen Analyten von dem Trägermaterial in eine bekannte Menge Untersuchungspuffer;
(f) Abtrennen des Trägermaterials vom Untersuchungspuffer; und
(g) quantitatives Bestimmen des Analyten,
**dadurch gekennzeichnet, dass** die wässrige Extraktionslösung im Überschuss 1 bis 50 mMol/L Ketocarbonsäure mit 1 bis 12 Kohlenstoffatomen enthält, die bei Umgebungsbedingungen mit Aminosäuren und Proteinen wasserlösliche hydratisierte Salze bilden, sowie übliche Puffersalze, Detergenzien, Solubilisierungsmittel, Komplexierungsmittel, Biozide, Adjuvanzien und Salze für die Einstellung der lonenstärke.

2. Verfahren nach Anspruch 1, wobei die Ketocarbonsäuren ausgewählt sind aus der Gruppe mit α-Ketomonocarbonsäuren, α-Ketodicarbonsäuren, β-Ketocarbonsäuren, β-Ketodicarbonsäuren, α-Ketopropionsäure (Brenztraubensäure), Acetessigsäure, Ketomalonsäure (Mesoxalsäure), Oxalessigsäure, Oxalbernsteinsäure, α-Ketoglutarsäure, Isocitrat, Oxohexansäure (Ketocapronsäure), Oxoheptansäure (Ketoönanthsäure), γ-Ketocarbonsäuren, Lävulinsäure, Phenylbrenztraubensäure, Ketoderivate und Ester der Terephthalsäure, sowie Mono- oder Diester der vorgenannten Ketocarbonäuren mit einem linearen oder verzweigten Alkohol mit 1 bis 6 Kohlenstoffatomen, Glyoxylsäure, Glyoxylsäureethylester, Brenztraubensäureethylester.

3. Verfahren nach Anspruch 1 oder Anspruch 2, wobei die Extraktionslösung 0,001 bis 10,0 Gew.%, bezogen auf das Gesamtgewicht der Zusammensetzung, vorzugsweise 0,005 bis 0,5 Gew.% ein oder mehrere Saccharide enthält, ausgewählt aus konservierendem Saccharid, Trehalose, Sucrose, Maltose.

4. Verfahren nach einem der Ansprüche 1 bis 3, wobei die Extraktionslösung ein oder mehrere Zusatzstoffe enthält, ausgewählt aus wasserlöslichem Farbstoff, pH-Indikator, Redox-Indikator, Fäulnisindikator, Methylenblau, Malachitgrün, Aromastoffe, Citronellal, Geraniol.

5. Verfahren nach einem der Ansprüche 1 bis 4, wobei der Träger Fasern aufweist, ausgewählt aus Cellulosefaser, Glasfaser oder Fasern aus Polyester, Polyethersulfon (PES), Polyamid (Nylon), Polypropylen, Polytetrafluorethylen (PTFE), Polycarbonat, Cellulosenitrat oder Celluloseacetat.

6. Verfahren nach einem der Ansprüche 1 bis 5, wobei die Extraktionslösung ein Fließmittel enthält, ausgewählt aus Polycarboxylat, Polycarboxylatether, Naphthalinsulfonat, Melaminsulfonat, Ligninsulfonat.

7. Verfahren nach einem der Ansprüche 1 bis 6, wobei ein Teil Probenmatrix mit 50 bis 250 Teilen, bevorzugt 80 bis 120 Teilen Extraktionslösung extrahiert wird.

8. Verfahren nach einem der Ansprüche 1 bis 7, wobei der feste Träger ausgelegt ist für eine definierte Aufnahme und zum Auftrocknen von 100 bis 250 µl wässriger Extraktionslösung und ein eventueller Überschuss an wässriger Extraktionslösung weggeleitet wird, so dass der darin enthaltene Analyt von der quantitativen Bestimmung ausgenommen ist.

9. Verfahren nach einem der Ansprüche 1 bis 8 zur quantitativen Bestimmung des Gehalts im Stuhl von Calprotectin (S100A8/S100A9; MRP8(MRP14); pankreatischer Elastase, Histamin, Laktoferrin, beta-Defensine (HBD-1, -2, -3, -5, -6), EDN (eosinophil-derived neurotoxin), Hämogloblin, Hämoglobin/Haptoglobin-Komplex.

10. Testbesteck, umfassend
(a) ein Stuhlaufnahmesystem mit einer bekannten Menge Extraktionslösung, enthaltend im Überschuss 1 bis 50 mMol/LKetocarbonsäure mit 1 bis 12 Kohlenstoffatomen, die bei Umgebungsbedingungen mit Aminosäuren und Proteinen wasserlösliche hydratisierte Salze bilden, sowie übliche Puffersalze, Detergenzien, Solubilisierungsmittel, Komplexierungsmittel, Biozide, Adjuvanzien sowie Salze für die Einstellung der lonenstärke sowie
(b) einen festen Träger,ausgelegt für eine definierte Aufnahme und zum Auftrocknen von 100 bis 250 µl wässriger Extraktionslösung, wobei ein eventueller Überschuss an wässriger Extraktionslösung weggeleitet wird, so dass der darin enthaltene Analyt von der quantitativen Bestimmung ausgenommen ist.

11. Testbesteck nach Anspruch 10, wobei der feste Träger über ein oder mehrere definierte Stege mit einer größeren Menge saugfähigen Fasermaterials verbunden ist, so dass überschüssige Flüssigkeit vom Träger abgeleitet wird.

12. Testbesteck nach Anspruch 10 oder Anspruch 11, ausgelegt für die Bestimmung des Gehalts im Stuhl von Calprotectin (S100A8/S100A9; MRP8(MRP14); pankreatischer Elastase, Histamin, Laktoferrin, beta-Defensin (HBD-1, -2, -3, -5, -6), EDN (eosinophil-derived neurotoxin), Hämogloblin, Hämoglobin/Haptoglobin-Komplex.

## Claims

1. A method for the preanalytical preparation of a sample of faeces, excrement or stool for the quantitative determination of a proteinaceous analyte, comprising the steps of:
(a) collecting the sample and transferring a known amount to a prepared vessel containing a known amount of aqueous extraction solution;
(b) dispersing and extracting the proteinaceous analyte from the sample matrix; and
(c) separating the aqueous extract from the solid matrix components and applying a known volume of the aqueous extract to a dry support material containing capillary material having capillary and chromatographic properties, such that the extract runs and spreads on the support material;
(d) drying the support material at ambient temperature, whereby the support with the dry extract provides a preservation and transport form;
(e) eluting the proteinaceous analyte from the support material into a known amount of known amount of assay buffer;
(f) separating the support material from the assay buffer; and
(g) quantitating the analyte,
**characterized in that** the aqueous extraction solution in excess of 1 to 50 mMol/L of a ketocarboxylic acid having from 1 to 12 carbon atoms, which is reacted with amino acids and proteins under ambient conditions, as well as conventional salts and conventional buffer salts, detergents, solubilizing agents, complexing agents, biocides, adjuvants and salts for adjusting the ionic strength.

2. The method according to claim 1, wherein the ketocarboxylic acids are selected from the group comprising α-keto monocarboxylic acids, α-keto dicarboxylic acids, β-ketocarboxylic acids, keto dicarboxylic acids, α-ketopropionic acid (pyruvic acid), acetoacetic acid, ketomalonic acid (mesoxalic acid), oxaloacetic acid, oxalsuccinic acid, α-ketoglutaric acid, isocitrate, oxohexanoic acid (ketocaproic acid), oxoheptanoic acid (ketoönanthic acid), γ-ketocarboxylic acids, levulinic acid, phenylpyruvic acid, keto derivatives and esters of terephthalic acid, and mono- or diesters of the aforementioned ketocarboxylic acids with a linear or branched alcohol having from 1 to 6 carbon atoms, glyoxylic acid, glyoxylic acid ethyl esters, pyruvic acid ethyl esters.

3. The method according to claim 1 or claim 2, wherein the extraction solution contains 0.001 to 10.0% by weight, based on the total weight of the composition, preferably 0.005 to 0.5% by weight of one or more saccharides selected from preservative saccharides, trehalose, sucrose, maltose.

4. The method according to any one of claims 1 to 3, wherein the extraction solution contains one or one or more additives selected from water-soluble dye, pH-indicator, redox-indicator, decay indicator, maltose, redox indicator, putrefaction indicator, methylene blue, malachite green, flavoring agents, citronellal, geraniol.

5. The method according to any one of claims 1 to 4, wherein the support comprises fibers, selected from cellulose fiber, glass fiber or fibers of polyester, polyether sulfone (PES), polyamide (nylon), polypropylene, polytetrafluoroethylene (PTFE), polycarbonate, cellulose nitrate or cellulose acetate.

6. The method according to any one of claims 1 to 5, wherein the extraction solution contains a flow agent selected from polycarboxylate, polycarboxylate ether, Naphthalene sulfonate, melamine sulfonate, lignin sulfonate.

7. The method according to any one of claims 1 to 6, wherein one part of the sample matrix is extracted with 50 to 250 parts, preferably 80 to 120 parts of extraction solution.

8. The method according to any one of claims 1 to 7, wherein the solid support is designed for a defined uptake and drying of 100 to 250 µl aqueous extraction solution and any excess of aqueous extraction solution is drained off so that the analyte contained therein is excluded from the quantitative determination.

9. The method according to any one of claims 1 to 8 for the quantitative determination of the content in stool of calprotectin (S100A8/S100A9; MRP8(MRP14); pancreatic elastase, histamine, lactoferrin, beta-defensins (HBD-1, -2, -3, -5, -6), EDN (eosinophil-derived neurotoxin), hemoglobin, hemoglobin/haptoglobin complex.

10. A test kit comprising.
(a) a stool collection system with a known amount of extraction solution, containing an excess from 1 to 50 mmol/L ketocarboxylic acid having from 1 to 12 carbon atoms, which, under ambient conditions, reacts with amino acids and proteins to form water-soluble hydrated salts, as well as usual buffer salts, detergents, solubilizing agents, complexing agents, solubilizers, solvents, solubilizing agents, complexing agents, biocides, adjuvants and salts for ionic strength adjustment, and
(b) a solid support, designed for a defined uptake and drying of 100 to 250 µl aqueous extraction solution, whereby a possible excess of aqueous extraction solution is conducted away so that the analyte contained therein is excluded from the quantitative determination.

11. The test kit according to claim 10, wherein the solid support is connected via one or more defined webs to a larger amount of absorbent fiber material, so that excess liquid is drained from the carrier.

12. The test kit according to claim 10 or claim 11, designed for the determination of the content in stool of calprotectin (S100A8/S100A9; MRP8(MRP14); pancreatic elastase, histamine, lactoferrin, beta-defensin (HBD-1, -2, -3, -5, -6), EDN (eosinophil- derived neurotoxin), hemoglobin, hemoglobin/haptoglobin complex.

## Revendications

1. Méthode de préparation préanalytique d'un échantillon de fèces, d'excréments ou de selles pour la détermination quantitative d'un analyte protéique, comprenant les étapes suivantes
(a) recueillir un échantillon et en transférer une quantité connue dans un récipient préparé contenant une quantité connue de solution d'extraction aqueuse ;
(b) disperser et extraire l'analyte protéique de la matrice de l'échantillon ; et
(c) séparer l'extrait aqueux des composants de la matrice solide et appliquer un volume connu de l'extrait aqueux à un support sec contenant un matériau capillaire ayant des propriétés capillaires et chromatographiques, de sorte que l'extrait s'écoule et s'étale sur le support ;
(d) séchage du support à température ambiante, le support contenant l'extrait sec constituant une forme de conservation et de transport ;
(e) élution de l'analyte protéique du support dans une quantité connue de tampon d'essai ;
(f) séparer le matériau de support du tampon d'essai ; et
(g) quantifier l'analyte,
**caractérisé en ce que** la solution aqueuse d'extraction contient en excès de 1 à 50 mMol/L d'un acide cétocarboxylique ayant de 1 à 12 atomes de carbone, qui réagit avec les acides aminés et les protéines dans des conditions ambiantes, ainsi que des sels conventionnels et des sels de tampon conventionnels, des détergents, des agents solubilisants, des agents complexant, des biocides, des adjuvants et des sels permettant d'ajuster la force ionique.

2. Méthode selon la revendication 1, dans laquelle les acides cétocarboxyliques sont choisis dans le groupe comprenant les acides α-cétomonocarboxyliques, les acides α-cétodicarboxyliques, les acides β-cétocarboxyliques, acides cétodicarboxyliques, acide α-cétopropionique (acide pyruvique), acide acétoacétique, acide cétomalonique (acide mésoxalique), acide oxaloacétique, acide oxalsuccinique, acide α-cétoglutarique, isocitrate, acide oxohexanoïque (acide cétocaproïque), acide oxoheptanoïque (acide cétoönanthic), acides γ-cétocarboxyliques, acide lévulinique, acide phénylpyruvique, dérivés céto et esters de l'acide téréphtalique, et les mono- ou diesters des acides cétocarboxyliques susmentionnés avec un alcool linéaire ou ramifié ayant de 1 à 6 atomes de carbone, l'acide glyoxylique, les esters éthyliques de l'acide glyoxylique, les esters éthyliques de l'acide pyruvique.

3. Procédé selon la revendication 1 ou la revendication 2, dans lequel la solution d'extraction contient de 0,001 à 10,0 % en poids, par rapport au poids total de la composition, de préférence de 0,005 à 0,5 % en poids d'un ou plusieurs saccharides choisis parmi les saccharides de conservation, le tréhalose, le saccharose, le maltose.

4. Procédé selon l'une quelconque des revendications 1 à 3, dans lequel la solution d'extraction contient un ou plusieurs additifs choisis parmi les colorants hydrosolubles, les indicateurs de pH, les indicateurs d'oxydoréduction, les indicateurs de putréfaction, le maltose, les indicateurs d'oxydoréduction, les indicateurs de putréfaction, le bleu de méthylène, le vert de malachite, les agents aromatisants, le citronellal, le géraniol.

5. Procédé selon l'une quelconque des revendications 1 à 4, dans lequel le support comprend des fibres, choisies parmi les fibres de cellulose, les fibres de verre ou les fibres de polyester, de polyéther sulfone (PES), de polyamide (nylon), de polypropylène, de polytétrafluoroéthylène (PTFE), de polycarbonate, de nitrate de cellulose ou d'acétate de cellulose.

6. Procédé selon l'une quelconque des revendications 1 à 5, dans lequel la solution d'extraction contient un agent d'écoulement choisi parmi le polycarboxylate, l'éther de polycarboxylate, le sulfonate de naphtalène, le sulfonate de mélamine, le sulfonate de lignine.

7. Méthode selon l'une des revendications 1 à 6, dans laquelle une partie de la matrice de l'échantillon est extraite avec 50 à 250 parties, de préférence 80 à 120 parties de solution d'extraction.

8. La méthode selon l'une des revendications 1 à 7, dans laquelle le support solide est conçu pour une absorption et un séchage défini de 100 à 250 µl de solution d'extraction aqueuse et tout excès de solution d'extraction aqueuse est drainé de sorte que l'analyte qu'il contient est exclu de la détermination quantitative.

9. Méthode selon l'une des revendications 1 à 8 pour la détermination quantitative de la teneur des selles en calprotectine (S100A8/S100A9 ; MRP8(MRP14) ; élastase pancréatique, histamine, lactoferrine, bêta-défensines (HBD-1, -2, -3, -5, -6), EDN (neurotoxine dérivée des éosinophiles), hémoglobine, complexe hémoglobine/haptoglobine.

10. Kit de test comprenant
(a) un système de collecte des selles contenant une quantité connue de solution d'extraction, contenant un excès de 1 à 50 mMol/L d'acide cétocarboxylique ayant de 1 à 12 atomes de carbone, qui, dans des conditions ambiantes, réagit avec les acides aminés et les protéines pour former des sels hydratés solubles dans l'eau, ainsi que des sels tampons habituels, des détergents, des agents solubilisants, des agents complexants, des solubilisants, des solvants, des agents solubilisants, des agents complexants, des biocides, des adjuvants et des sels pour l'ajustement de la force ionique, et
(b) un support solide conçu pour absorber et sécher de manière définie 100 à 250 µl de solution d'extraction aqueuse, de sorte qu'un éventuel excès de solution d'extraction aqueuse soit éliminé et que l'analyte qu'il contient soit exclu de la détermination quantitative.

11. Le kit de test selon la revendication 10, dans lequel le support solide est relié par une ou plusieurs bandes définies à une plus grande quantité de matériau fibreux absorbant, de sorte que l'excès de liquide est évacué du support.

12. Le kit de test selon la revendication 10 ou la revendication 11, conçu pour la détermination du contenu des selles en calprotectine (S100A8/S100A9 ; MRP8(MRP14) ; élastase pancréatique, histamine, lactoferrine, bêta-défensine (HBD-1, -2, -3, -5, -6), EDN (neurotoxine dérivée des éosinophiles), hémoglobine, complexe d'hémoglobine/haptoglobine.
